# DEMANDE DE BREVET EUROPEEN

(11) **EP 0 919 239 A1**
(43) Date de publication de la demande: **02.06.1999**
(21) Numéro de dépôt: 98450017.3
(22) Date de dépôt: 10.11.1998
(51) Int. Cl.: A61K 35/72, A61K 35/02, A61K 33/14

(54) **Composition pour le traitement des gastro-entérites aigues, comprenant une solution de réhydratation avec un composé à action synérgique**

(30) Priorité: 12.11.1997 FR 9714391
(71) Demandeur: Investigations therapeutiques essais cliniques services société à Responsabilité Limitée, 33560 Sainte Eulalie (FR)
(72) Inventeur: Auzerie, Jack, 33560 Sainte Eulalie (FR)
(74) Mandataire: Thébault, Jean-Louis

(57) **Abrégé**

L'objet de l'invention est une composition de traitement de gastro-entérites comprenant la combinaison d'une solution de réhydratation avec au moins un composé ayant une action de synergie, choisi parmi les antiseptiques et/ou les prébiotiques et/ou les modificateurs du transit intestinal et/ou les terres.

L'invention concerne aussi le procédé de fabrication de cette solution et une solution particulière obtenue.

## Description

L'invention concerne le traitement des gastro-entérites aiguës à l'aide d'une solution obtenue à partir d'une composition également objet de la présente demande ainsi que le procédé de fabrication d'une telle composition.

En effet, on connaît le problème des gastro-entérites aiguës qui sont relativement difficiles à traiter dans les pays du tiers monde mais aussi dans les pays où la médecine est fortement avancée.

En effet, la déshydratation est une complication majeure des gastro-entérites chez les nourrissons et les jeunes enfants et actuellement, dans les pays à médecine avancée, on a réussi à diminuer les risques de décès liés à cette déshydratation mais en administrant des injections par voie intraveineuse de produits de réhydratation en milieu hospitalier.

C'est ainsi que dans la demande de brevet français N° 83 20861, il est indiqué une composition et son mode d'administration par voie orale en vue de réhydrater un enfant atteint d'une gastro-entérite, notamment aiguë, d'origine virale, ou infecté par le choléra lors d'une épidémie, ce qui supprime, pour de nombreux cas, les besoins d'hospitalisation.

On sait que la diarrhée aiguë peut se définir à partir de notions physiopathologiques, à savoir une malabsorption de l'eau qui entre chaque jour en grande quantité dans l'intestin, à laquelle vient s'ajouter les sécrétions d'origine digestive, stimulées par l'ingestion des aliments.

Ce volume de liquides est ré-absorbé par l'intestin grêle et le colon ne laissant dans les selles, en régime normal, qu'un faible volume desdits liquides. La diarrhée est un excès d'eau fécale lié à une mauvaise ré-absorption ou une sécrétion anormale.

Des traitements étiologiques, comme la soupe de carottes ont été longtemps administrés sans que la preuve puisse être faite de l'efficacité mais de tels traitements ont plutôt tendance à masquer les conséquences.

Néanmoins, compte tenu des difficultés d'administration de solutés par voie intraveineuse en milieu hospitalier, la voie du traitement nutritionnel paraît la plus adaptée.

Le glucose notamment paraît agir sur la toxine cholérique tout en améliorant le système d'absorption du sodium dont on sait qu'il est lié aux mouvements de l'eau dans le corps.

Aussi, des solutés pour une administration par voie orale ont été mis au point et notamment dans la demande de brevet ci-dessus mentionnée, la composition d'un tel soluté comprenant :
- 10 à 20 g de glucose,
- 10 à 40 g de saccharose,
- 5 à 40 méq de citrate,
- 20 à 75 méq de chlorure,
- 0 à 50 méq de potassium, et
- 40 à 75 méq de sodium,
l'ensemble ayant de préférence une osmolalité comprise entre 280 et 320 m.osmol.

Une telle composition permet d'obtenir de bons effets de réhydratation et en plus, le goût n'est pas rendu désagréable par la forte teneur en bicarbonate, si bien que l'acceptabilité de la part des nourrissons est améliorée, ce qui est un progrès important quand on sait que de tels solutés sont les seuls moyens d'agir pour certaines populations. Le tampon par le citrate permet de remplacer le bicarbonate en évitant de la même façon l'acidose.

On peut noter que chez l'adulte, les problèmes de réhydratation sont aussi importants mais les conséquences sont moins significatives si bien que la réhydratation est rarement prise en compte et cette thérapeutique de réhydratation est rarement proposée ou imposée aux adultes.

Néanmoins, les symptômes généraux présentés sont identiques à ceux du nourrisson et montrent qu'il y a bien déshydratation, plus particulièrement dans le cas de la diarrhée du voyageur.

La présente invention propose une composition de traitement de gastro-entérites combinant une solution de réhydratation avec au moins un composé ayant une action de synergie, choisi notamment parmi les antiseptiques intestinaux et/ou les probiotiques et/ou les modificateurs du transit intestinal et/ou les terres incluant les argiles.

1/ Dans le cas d'un antiseptique intestinal, on sait qu'il a une action sur l'agent étiologique et un effet curatif mais que, utilisé seul, il a un effet moins complet puisque la réhydratation nécessaire qui doit être associée au traitement curatif n'est bien souvent pas mise en oeuvre ou au contraire mise en oeuvre en milieu hospitalier par intraveineuse avec le chapelet de contraintes qu'il engendre. On comprend que, dans le cas où l'antiseptique est combiné avec la solution de réhydratation, on supprime les inconvénients liés à la prise supplémentaire d'un traitement séparé, à la non prescription soit de l'antiseptique soit du traitement de réhydratation suivant qu'il s'agit d'un nourrisson ou d'un adulte et à l'étalement dans le temps des actions des différents composés, supprimant l'effet de synergie.

Un tel antiseptique intestinal peut être du *Nifuroxazide.*

La posologie est alors liée à la fréquence des prises de la solution de réhydratation et adaptée en conséquence.

Une composition particulièrement convenable comprend par exemple pour un adulte :
- 909,2 mg de *Nifuroxazide,*
- 10 à 20 g de glucose,
- 10 à 40 g de saccharose,
- 5à 40 méq de citrate,
- 20 à 75 méq de chlorure,
- 0 à 50 méq de potassium, et
- 40 à 75 méq de sodium,
l'osmolalité étant comprise entre 150 et 350 m.osml.

Plus particulièrement, elle comprend :
- 909,2 mg de *Nifuroxazide,*
- 16 g de glucose,
- 20 g de saccharose,
- 23,1 méq de citrate,
- 26,5 méq de chlorure,
- 26 méq de potassium, et
- 67,8 méq de sodium,
l'osmolalité étant de l'ordre de 280 m.osmol.

Le procédé de fabrication de cette composition consiste à mélanger sous forme de poudre :
- 909,2 mg de *Nifuroxazide,*
- 16 g de glucose,
- 20 g de saccharose,
- 2 g de chlorure de potassium, et
- 6 g de citrate de potassium.

L'invention a aussi pour objet une solution de traitement d'une gastro-entérite qui comprend dans un litre d'eau :
- 909,2 mg de *Nifuroxazide,*
- 10 à 20 g de glucose,
- 10 à 40 g de saccharose,
- 5à 40 méq de citrate,
- 20 à 75 méq de chlorure,
- 0 à 50 méq de potassium, et
- 40 à 75 méq de sodium,
avec une osmolalité de 150 à 350 m.osmol.

De façon plus précise, la solution comprend :
- 909,2 mg de *Nifuroxazide,*
- 16 g de glucose,
- 20 g de saccharose,
- 23,1 méq de citrate,
- 26,5 méq de chlorure,
- 26 méq de potassium, et
- 67,8 méq de sodium,
l'osmolalité étant de l'ordre de 280 m.osmol.

Une telle solution peut être administrée par voie orale. Le conditionnement peut se faire sous forme de poudre en vrac en grosse quantité pour les antennes médicales ou le milieu hospitalier et en sachets unitaires pour une consommation familiale.

La dose est adaptée au volume d'un verre, par exemple 200 mg pour un verre de 220 ml.

2/ Dans le cas d'un prébiotique, on sait qu'il a une action sur l'agent étiologique et un effet curatif mais que, utilisé seul, il a un effet moins complet puisque la réhydratation nécessaire qui doit être associée au traitement curatif n'est bien souvent pas mise en oeuvre ou au contraire mise en oeuvre en milieu hospitalier avec le chapelet de contraintes qu'il engendre. On comprend que, dans le cas où le prébiotique est combiné avec la solution de réhydratation, on supprime les inconvénients liés à la prise supplémentaire d'un traitement séparé, à la non prescription soit du prébiotique soit du traitement de réhydratation et à l'étalement dans le temps des actions des différents composés, supprimant l'effet de synergie.

Un tel probiotique peut être *Saccharomyces boulardii*

La posologie est alors liée à la fréquence des prises de la solution de réhydratation et adaptée en conséquence.

Une composition particulièrement convenable comprend par exemple pour un adulte :
- 256,84 mg de *Saccharomyces boulardii,*
- 10 à 20 g de glucose,
- 10 à 40 g de saccharose,
- 5à 40 méq de citrate,
- 20 à 75 méq de chlorure,
- 0 à 50 méq de potassium, et
- 40 à 75 méq de sodium,
l'osmolalité étant comprise entre 150 et 350 m.osml.

Plus particulièrement, elle comprend :
- 256,84 mg de *Saccharomyces boulardii,*
- 16 g de glucose,
- 20 g de saccharose,
- 23,1 méq de citrate,
- 26,5 méq de chlorure,
- 26 méq de potassium, et
- 67,8 méq de sodium,
l'osmolalité étant de l'ordre de 280 m.osmol.

Le procédé de fabrication de cette composition consiste à mélanger sous forme de poudre :
- 256,84 mg de *Saccharomyces boulardii,*
- 16 g de glucose,
- 20 g de saccharose,
- 2 g de chlorure de potassium, et
- 6 g de citrate de potassium.

L'invention a aussi pour objet une solution de traitement d'une gastro-entérite qui comprend dans un litre d'eau :
- 256,84 mg de *Saccharomyces boulardii,*
- 10 à 20 g de glucose,
- 10 à 40 g de saccharose,
- 5à 40 méq de citrate,
- 20 à 75 méq de chlorure,
- 0 à 50 méq de potassium, et
- 40 à 75 méq de sodium,
avec une osmolalité de 150 à 350 m.osmol.

De façon plus précise, la solution comprend :
- 256,84 mg de *Saccharomyces boulardii,*
- 16 g de glucose,
- 20 g de saccharose,
- 23,1 méq de citrate,
- 26,5 méq de chlorure,
- 26 méq de potassium, et
- 67,8 méq de sodium,
l'osmolalité étant de l'ordre de 280 m.osmol.

Une telle solution peut être administrée par voie orale. Le conditionnement peut se faire sous forme de poudre en vrac en grosse quantité pour les antennes médicales ou le milieu hospitalier et en sachets unitaires pour une consommation familiale. Plus particulièrement, dans le cas des nourrissons, la dose est adaptée au volume d'un biberon, par exemple 56,5 g pour un volume de 220 ml, pour un nombre de 4 à 8 biberons par jour.

3/ Dans le cas d'un modificateur du transit intestinal, on sait qu'il a un effet curatif mais que, utilisé seul, il a un effet moins complet puisque la réhydratation nécessaire qui doit être associée au traitement curatif n'est bien souvent pas mise en oeuvre ou au contraire mise en oeuvre en milieu hospitalier avec le chapelet de contraintes qu'il engendre. On comprend que, dans le cas où le modificateur de transit intestinal est combiné avec la solution de réhydratation, on supprime les inconvénients liés à la prise supplémentaire d'un traitement séparé, à la non prescription soit du modificateur du transit intestinal soit du traitement de réhydratation et à l'étalement dans le temps des actions des différents composés, supprimant l'effet de synergie.

Un tel modificateur du transit intestinal peut être du *Lopéramide.*

La posologie est alors liée à la fréquence des prises de la solution de réhydratation et adaptée en conséquence.

Une composition particulièrement convenable comprend par exemple pour un adulte :
- 9,08 mg de *Lopéramide,*
- 10 à 20 g de glucose,
- 10 à 40 g de saccharose,
- 5à 40 méq de citrate,
- 20 à 75 méq de chlorure,
- 0 à 50 méq de potassium, et
- 40 à 75 méq de sodium,
l'osmolalité étant comprise entre 150 et 350 m.osml.

Plus particulièrement, elle comprend :
- 9,08 mg de *Lopéramide,*
- 16 g de glucose,
- 20 g de saccharose,
- 23,1 méq de citrate,
- 26,5 méq de chlorure,
- 26 méq de potassium, et
- 67,8 méq de sodium,
l'osmolalité étant de l'ordre de 280 m.osmol.

Le procédé de fabrication de cette composition consiste à mélanger sous forme de poudre :
- 9,08 mg de *Lopéramide,*
- 16 g de glucose,
- 20 g de saccharose,
- 2 g de chlorure de potassium, et
- 6 g de citrate de potassium.

L'invention a aussi pour objet une solution de traitement d'une gastro-entérite qui comprend dans un litre d'eau :
- 9,08 mg de *Lopéramide,*
- 10 à 20 g de glucose,
- 10 à 40 g de saccharose,
- 5à 40 méq de citrate,
- 20 à 75 méq de chlorure,
- 0 à 50 méq de potassium, et
- 40 à 75 méq de sodium,
avec une osmolalité de 150 à 350 m.osmol.

De façon plus précise, la solution comprend :
- 9,08 mg de *Lopéramide,*
- 16 g de glucose,
- 20 g de saccharose,
- 23,1 méq de citrate,
- 26,5 méq de chlorure,
- 26 méq de potassium, et
- 67,8 méq de sodium,
l'osmolalité étant de l'ordre de 280 m.osmol.

Une telle solution peut être administrée par voie orale. Le conditionnement peut se faire sous forme de poudre en vrac en grosse quantité pour les antennes médicales ou le milieu hospitalier et en sachets unitaires pour une consommation familiale. Plus particulièrement, la dose est adaptée au volume d'un verre, par exemple 2 mg de *Lopéramide* pour un verre de 220 ml.

4/ Dans le cas d'argiles ou plus généralement de terres, on sait qu'elles ont une action sur l'agent étiologique et un effet curatif mais que, utilisées seules, elles ont un effet moins complet puisque la réhydratation nécessaire qui doit être associée au traitement curatif n'est bien souvent pas mise en oeuvre ou au contraire mise en oeuvre en milieu hospitalier avec le chapelet de contraintes qu'il engendre. On comprend que, dans le cas où les terres sont combinées avec la solution de réhydratation, on supprime les inconvénients liés à la prise supplémentaire d'un traitement séparé, à la non prescription soit des argiles ou terres soit du traitement de réhydratation et à l'étalement dans le temps des actions des différents composés, supprimant l'effet de synergie.

Une telle composition, comprenant au moins une terre, peut être choisie parmi la *Diosmectite* ou l'*Attapulgite* en particulier celle de Mormoiron sous forme activée.

La posologie est alors liée à la fréquence des prises de la solution de réhydratation et adaptée en conséquence.

Une composition particulièrement adaptée comprend par exemple pour un litre de solution :
- 4 g de *Diosmectite* ou d'*Attapulgite* ou d'un mélange des deux,
- 10 à 20 g de glucose,
- 10 à 40 g de saccharose,
- 5à 40 méq de citrate,
- 20 à 75 méq de chlorure,
- 0 à 50 méq de potassium, et
- 40 à 75 méq de sodium,
l'osmolalité étant comprise entre 150 et 350 m.osml.

Plus particulièrement, elle comprend :
- 4 g de *Diosmectite* ou d'*Attapulgite* ou d'un mélange des deux,
- 16 g de glucose,
- 20 g de saccharose,
- 23,1 méq de citrate,
- 26,5 méq de chlorure,
- 26 méq de potassium, et
- 67,8 méq de sodium,
l'osmolalité étant de l'ordre de 280 m.osmol.

Le procédé de fabrication de cette composition consiste à mélanger sous forme de poudre :
- 4 g de *Diosmectite* ou d'*Attapulgite* ou d'un mélange des deux,
- 16 g de glucose,
- 20 g de saccharose,
- 2 g de chlorure de potassium, et
- 6 g de citrate de potassium.

L'invention a aussi pour objet une solution de traitement d'une gastro-entérite qui comprend dans un litre d'eau :
- 4 g de *Diosmectite* ou d'*Attapulgite* ou d'un mélange des deux,
- 10 à 20 g de glucose,
- 10 à 40 g de saccharose,
- 5à 40 méq de citrate,
- 20 à 75 méq de chlorure,
- 0 à 50 méq de potassium, et
- 40 à 75 méq de sodium,
avec une osmolalité de 150 à 350 m.osmol.

De façon plus précise, la solution comprend :
- 4 g de *Diosmectite* ou d'*Attapulgite* ou d'un mélange des deux
- 16 g de glucose,
- 20 g de saccharose,
- 23,1 méq de citrate,
- 26,5 méq de chlorure,
- 26 méq de potassium, et
- 67,8 méq de sodium,
l'osmolalité étant de l'ordre de 280 m.osmol.

Une telle solution peut être administrée par voie orale. Le conditionnement peut se faire sous forme de poudre en vrac en grosse quantité pour les antennes médicales ou le milieu hospitalier et en sachets unitaires pour une consommation familiale. Plus particulièrement, dans le cas des nourrissons, la dose est adaptée au volume d'un biberon, par exemple 0,87 g pour un biberon de 220 ml, dans le cas de 4 à 8 biberons par jour.

De telles compositions et solutions facilitent la prise par les nourrissons en une seule action tandis que la combinaison permet aussi d'imposer à l'adulte la réhydratation qui est omise, facilitant ainsi l'obtention de la fin des troubles et la remise en fonction du système de ré-absorption limitant la quantité d'eau dans les selles.

Les adultes ne peuvent pas séparer les composés pour ne prendre que ceux qu'ils jugent utiles si bien que, malgré eux, l'effet de synergie est obtenu, raccourcissant les délais de retour à la normale.

## Revendications

1. Composition de traitement de gastro-entérites comprenant la combinaison d'une solution de réhydratation avec au moins un composé ayant une action de synergie, choisi parmi les anti-septiques et/ou les prébiotiques et/ou les modificateurs du transit intestinal et/ou les terres.

2. Composition de traitement selon la revendication 1, caractérisée en ce que la solution de réhydratation comprend :
- 10 à 20 g de glucose,
- 10 à 40 g de saccharose,
- 5à 40 méq de citrate,
- 20 à 75 méq de chlorure,
- 0 à 50 méq de potassium, et
- 40 à 75 méq de sodium,
l'osmolalité étant comprise entre 150 et 350 m.osml.

3. Composition selon la revendication 1 ou 2, caractérisée en ce que l'un des composés ayant un effet de synergie est un anti-septique, le *Nifuroxazide.*

4. Composition selon la revendication 3, caractérisée en ce que l'antiseptique, le *Nifuroxazide,* est en quantité de l'ordre de 900 mg (pour un litre de solution).

5. Composition selon la revendication 1 ou 2, caractérisée en ce que l'un des composés ayant un effet de synergie est un prébiotique, *Saccharomyces boulardii.*

6. Composition selon la revendication 2 et 5, caractérisée en ce que le prébiotique *Saccharomyces boulardii* est en quantité de l'ordre de 250 mg (pour un litre de solution).

7. Composition selon la revendication 1 ou 2, caractérisée en ce que l'un des composés ayant un effet de synergie est un régulateur du transit intestinal, le *Lopéramide.*

8. Composition selon la revendication 2 et 7, caractérisée en ce que le régulateur du transit intestinal, le *Lopéramide,* est en quantité de l'ordre de 9 mg.

9. Composition selon la revendication 1 ou 2, caractérisée en ce que l'un des composés ayant un effet de synergie est une terre du type argile, la *Diosmectite* ou l'*Attapulgite.*

10. Composition selon la revendication 2 et 7, caractérisée en ce que la terre du type argile, la *Diosmectite* ou l'*Attapulgite,* est en quantité de l'ordre de 4 g.

11. Procédé de fabrication de la composition selon l'une quelconque des revendications 1 à 10, caractérisé en ce qu'il consiste à mélanger sous forme de poudres les différents composés en quantités correspondantes.

12. Procédé selon la revendication 11, caractérisé en ce que, dans le cas d'une composition comprenant du *Nifuroxazide,* on mélange à l'état de poudre :
- 902,2 mg de *Nifuroxazide,*
- 16 g de glucose,
- 20 g de saccharose,
- 2 g de chlorure de potassium, et
- 6 g de citrate de potassium.

13. Procédé selon la revendication 11, caractérisé en ce que, dans le cas d'une composition comprenant le prébiotique *Saccharomyces boulardii,* on mélange à l'état de poudre :
- 256,84 mg de *Saccharomyces boulardii,*
- 16 g de glucose,
- 20 g de saccharose,
- 2 g de chlorure de potassium, et
- 6 g de citrate de potassium.

14. Procédé selon la revendication 11, caractérisé en ce que, dans le cas d'une composition comprenant le régulateur du transit intestinal, le *Lopéramide,* on mélange à l'état de poudre :
- 9,08 mg de *Lopéramide,*
- 16 g de glucose,
- 20 g de saccharose,
- 2 g de chlorure de potassium, et
- 6 g de citrate de potassium.

15. Procédé selon la revendication 11, caractérisé en ce que, dans le cas d'une composition comprenant de la terre du type argile, la *Diosmectite* ou l'*Attapulgite,* on mélange à l'état de poudre :
- 4 g de *Diosmectite* ou d'*Attapulgite,*
- 16 g de glucose,
- 20 g de saccharose,
- 2 g de chlorure de potassium, et
- 6 g de citrate de potassium.

16. Solution pour le traitement de gastro-entérites comprenant la composition selon les revendications 4 et 12, caractérisée en ce que l'on mélange dans un litre d'eau :
- 909,2 mg de *Nifuroxazide,*
- 16 g de glucose,
- 20 g de saccharose,
- 2 g de chlorure de potassium, et
- 6 g de citrate de potassium,
l'osmolalité étant comprise entre 150 et 350 m.osml.

17. Solution pour le traitement de gastro-entérites comprenant la composition selon les revendications 6 et 13, caractérisée en ce que l'on mélange dans un litre d'eau :
- 256,84 mg de *Saccharomyces boulardii,*
- 16 g de glucose,
- 20 g de saccharose,
- 2 g de chlorure de potassium, et
- 6 g de citrate de potassium,
l'osmolalité étant comprise entre 150 et 350 m.osml.

18. Solution pour le traitement de gastro-entérites comprenant la composition selon les revendications 8 et 14, caractérisée en ce que l'on mélange dans un litre d'eau :
- 9,08 mg de *Lopéramide,*
- 16 g de glucose,
- 20 g de saccharose,
- 2 g de chlorure de potassium, et
- 6 g de citrate de potassium,
l'osmolalité étant comprise entre 150 et 350 m.osml.

19. Solution pour le traitement de gastro-entérites comprenant la composition selon les revendications 10 et 15, caractérisée en ce que l'on mélange dans un litre d'eau :
- 4 g de *Diosmectite* ou d'*Attapulgite,*
- 16 g de glucose,
- 20 g de saccharose,
- 2 g de chlorure de potassium, et
- 6 g de citrate de potassium,
l'osmolalité étant comprise entre 150 et 350 m.osml.
